# EUROPEAN PATENT APPLICATION

(11) **EP 3 699 167 A1**
(43) Date of publication of application: **26.08.2020**
(21) Application number: 19305216.4
(22) Date of filing: 22.02.2019
(51) Int. Cl.: C07C 67/36, C07C 69/76

(54) **PROCESS FOR PREPARING METHYL 1-BENZOSUBERONE-7-CARBOXYLATE**

(71) Applicant: Sanofi, 75008 Paris (FR)
(72) Inventor: TAMMANA, Rajesh, 600057 Chennai (IN); KUMAR GANAPATHY, Ashok, 600057 Chennai (IN); WEHREY, Christian, 75008 Paris (FR)
(74) Representative: Nony

(57) **Abstract**

Herein is provided a process for preparing compound (A5) 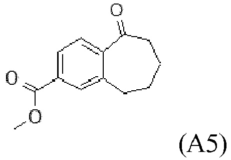 characterized in that a compound of formula (e) 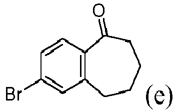 is alkoxycarbonylated to prepare compound (A5).

Herein is also provided a process for the preparation of compound (1) or one of its pharmaceutically acceptable salts 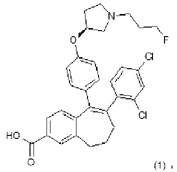 characterized in that it comprises the step of using a compound (A5) as prepared by the process according to the present invention.

## Description

Herein is provided a novel process for the preparation of methyl 1-benzosuberone-7-carboxylate (hereafter "compound (A5)") also named as methyl 5-oxo-6,7,8,9-tetrahydro-5*H*-benzo[7]annulene-2-carboxylate. Said compound is an intermediate for the preparation of 6-(2,4-dichlorophenyl)-5-[4-[(3S)-1-(3-fluoropropyl)pyrrolidin-3-yl]oxyphenyl]-8,9-dihydro-7*H*-benzo[7]annulene-2-carboxylic acid.

Herein is also provided a novel process for the preparation of 6-(2,4-dichlorophenyl)-5-[4-[(3S)-1-(3-fluoropropyl)pyrrolidin-3-yl]oxyphenyl]-8,9-dihydro-7*H-*benzo[7]annulene-2-carboxylic acid and a salt thereof.

6-(2,4-dichlorophenyl)-5-[4-[(3S)-1-(3-fluoropropyl)pyrrolidin-3-yl]oxyphenyl]-8,9-dihydro-7*H*-benzo[7]annulene-2-carboxylic acid (hereafter "compound (1)"), depicted below, is a selective estrogen receptor degrader (SERD) which has estrogen receptor antagonist properties and accelerates the proteasomal degradation of the estrogen receptor. It may be used in particular as anticancer agent. This compound is disclosed in the patent application WO 2017/140669.

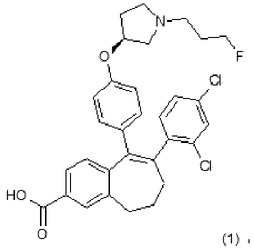

For active ingredients in medicinal products and their synthesis intermediates there is always a need to find new synthesis routes more adapted for industrial implementation.

The benzosuberone derivative as defined above is used in the synthetic process as described in WO 2017/140669 for preparing 6-(2,4-dichlorophenyl)-5-[4-[(3S)-1-(3-fluoropropyl)pyrrolidin-3-yl]oxyphenyl]-8,9-dihydro-7*H*-benzo[7]annulene-2-carboxylic acid and was already named compound (A5). Still in WO 2017/140669, said compound (A5) is obtained by using as a starting compound 2-methoxy-6,7,8,9-tetrahydro-5*H-*benzo[7]annulen-5-one commercially available *via* the preparation of intermediate 2-hydroxy-6,7,8,9-tetrahydro-5*H*-benzo[7]annulen-5-one (compound (A2)) and then of intermediate 5-oxo-6,7,8,9-tetrahydro-5*H*-benzo[7]annulen-2-yltrifluoromethanesulfonate (compound (A4)), i.e. within 3 steps.

However, said route of synthesis presents numerous drawbacks, in particular in terms of large number of manufacturing steps and used purification method, and namely column chromatography, that is not scalable. More particularly, as it comes out from said known process, obtaining compound (A5) requires three steps starting from 2-methoxy-6,7,8,9-tetrahydro-5*H*-benzo[7]annulen-5-one which itself is more or less obtained in the same number of steps than compound of formula (e) as defined herein after. In other words, the synthetic route known in the art for obtaining compound (A5) needs two additional steps, namely demethylation followed by triflation, in comparison to the process according to the present invention. Moreover, said compound (A5) is afforded as an oil, which is difficult to handle on scale. In other words, in comparison to the obtention of compound (A5) according to WO 2017/140669, the present invention, starting from compound of formula (e), provides an advantageous decrease of manufacturing steps and a scalable process.

It has then been stated the following advantages in comparison to said known process: straight forward, cost effective, robust, scalable, high quality and high yielding.

As used herein, the term "ambient temperature" or "room temperature" refers to a temperature ranging from 23°C to 35°C, more particularly from 25°C to 35°C.

Herein is therefore provided an improved process for preparing compound (A5) 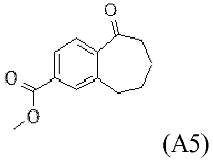 characterized in that a compound of formula (e) 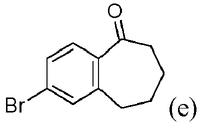 is alkoxycarbonylated to prepare compound (A5).

Said process step corresponds to step (iv) as more detailed herein after.

It is a constant need to control the purity of key intermediates. The present invention is also intended to provide a method for preparing a compound (A5) with controlled purity. The present invention achieves this need by providing a manufacturing process in four steps, to have a better control on the impurity profile of compound (A5). Accordingly, said compound (A5) may be manufactured with high quality (>99.5% purity by HPLC) as a solid, in the framework of the present invention.

As a further advantage, the accessibility as a commercial product, and low cost of the starting material 3-bromobenzaldehyde may be cited.

Compound of formula (e) may be prepared by cyclizing of compound of formula (d)

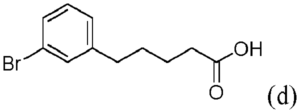

Said process step correponds to step **(iii)** as more detailed herein after.

Compound (d) may be prepared by hydrogenating compound of formula (c)

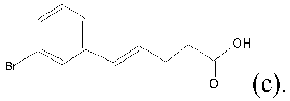

Said process step corresponds to step **(ii)** as more detailed herein after.

Compound of formula (c) may be prepared by reacting a compound of formula (a) 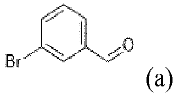 with a compound of formula 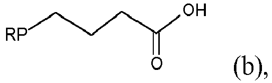 of formula 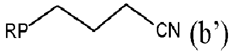 or of formula 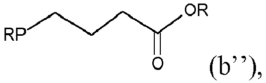 wherein RP means a X⁻P⁺R'₃ group or a P(=O)(OR")₂, respectively under Wittig or Wittig-Horner conditions, R means a (C₁-C₆)alkyl group, R' means a (C₁-C₃)alkyl group or a phenyl group, R" means a (C₁-C₃)alkyl group and X is an halogen atom, and in particular a bromine atom, the carbon in alpha of the phosphorus atom being a carbanion.

Said process step corresponds to step **(i)** as more detailed herein after.

Herein is further provided an improved process for preparing a compound (A5) as defined above
characterized in that it comprises:
- a step **(i)**: reacting a compound of formula (a) 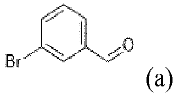 with a compound of formula 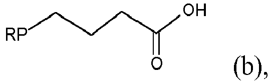 of formula 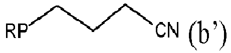 or of formula 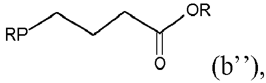 wherein RP means a X⁻P⁺R'₃ group or a P(=O)(OR")₂, respectively under Wittig or Wittig-Horner conditions, R means a (C₁-C₆)alkyl group, R' means a (C₁-C₃)alkyl group or a phenyl group, R" means a (C₁-C₃)alkyl group and X is an halogen atom, and in particular a bromine atom, the carbon in alpha of the phosphorus atom being a carbanion,
   to prepare compound of formula (c) 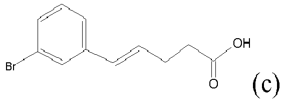
- a step **(ii)**: hydrogenating compound of formula (c) to prepare compound of formula (d) 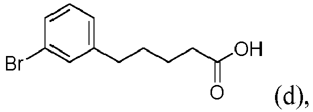
- a step **(iii)**: cyclizing of compound of formula (d) to prepare compound of formula (e) 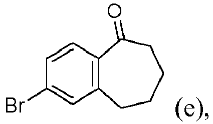 and
- a step **(iv)**: alkoxycarbonylating of compound of formula (e) to prepare compound (A5).

Compound of formula (a) is also named as 3-bromobenzaldehyde.

Compound of formula (b) maybe the compound named as (3-carboxypropyl)triphenylphosphonium bromide as detailed herein after.

Compound of formula (c) is also named as 5-(3-bromophenyl)pent-4-enoic acid.

Compound of formula (d) is also named as 5-(3-bromophenyl)pentanoic acid.

Compound of formula (e) is also named as 2-bromo-6,7,8,9-tetrahydro-5*H-*benzo[7]annulen-5-one or 7-Bromo-1-benzosuberone.

Herein is also provided a novel process for the preparation of compound (1) or one of its pharmaceutically acceptable salts 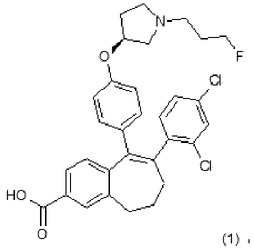 characterized in that it comprises the step of using a compound (A5) as prepared by the process according to the present invention.

Herein is further provided a process for preparing a compound (1) as defined above, further comprising the step of preparing a pharmaceutical composition containing such compound (1), with pharmaceutically acceptable excipients.

The subsequent steps following the preparation of compound (A5) for preparing compound (1) and its pharmaceutically acceptable salts may be performed according to steps as disclosed in WO 2017/140669 or any other suitable route of synthesis.

The process according to the present invention may also be used for preparing pharmaceutically acceptable salts of compound (1).

In the context of the present invention, the term:
- "halogen" is understood to mean chlorine, fluorine, bromine, or iodine, and in particular denotes chlorine, fluorine or bromine, and more particularly a bromine atom,
- "(C₁-Cₓ)alkyl" as used herein refers to a linear or branched C₁-Cₓ alkyl, that may in particular be (C₁-C₃)alkyl or (C₁-C₆)alkyl. Examples are, but are not limited to, methyl, ethyl, n-propyl, isopropyl, n-butyl, tert-butyl, pentyl and the like, and
- "pharmaceutically acceptable" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

### Step (i)

Step (i) may be performed according to Wittig or Wittig-Horner conditions.

According to a particular embodiment, step (i) may be carried out in the presence of a base. Said base may be selected from Li/Na/K alkoxides, and more particularly sodium methoxide, Li/Na/K bis(trimethylsilyl)amide (Li/Na/KHMDS), Li/Na/K diisopropylamide (Li/Na/K DA), NaH, tBuOK and n-BuLi and the like and may in particular be sodium methoxide.

Still according to said particular embodiment, said step (i) may be carried out in a solvent, and more particularly an organic solvent which may be selected from tetrahydrofuran, methylated tetrahydrofuran, ethers such as dimethylether (DME), toluene, dimethyl sulfoxide (DMSO) and dichloromethane and the like, as well as their mixture, each of said solvent being optionally mixed with methanol, and may in particular be tetrahydrofuran optionally mixed with methanol.

Step (i) may be carried out over a wide temperature range which does not cause side reactions, but may be carried out at a reaction temperature ranging from 0 °C to 70°C, in particular from 25°C to 65°C, and most particularly from 40 to 45°C.

Step (i) may be carried out for 1 to 48 hours, in particular for 5 to 24 hours, and even more particularly for 8 to 12 hours.

The molar ratio of the compound of formula (a) vs. the compound of formula (b) may in particular range between 0.5 and 1.25, in particular between 0.6 and 1.0, and more particularly between 0.7 and 0.9.

Said step (i) may be followed by any purification step, in particular dedicated to remove triphenylphosphine oxide (Ph₃P=O). The pH of the aqueous layer may be adjusted to about 1, for example with hydrochloric acid and said aqueous layer may then be extracted, for example with toluene. The obtained organic phase may then be washed, for example with a sodium chloride solution. During said step (i), solvents may be removed by additional distillation steps or concentration under reduced pressure.

Compound of formula (b) may in particular be chosen from (3-carboxypropyl)triphenylphosphonium bromide of formula (b1) 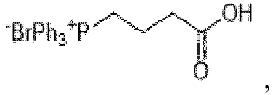 in particular when used in the Wittig conditions.

Compound of formula (b) may in particular be further selected from 4-(diethoxyphosphoryl)butanoic acid, 4-(dimethoxythiophosphoryl)butanoic acid and the like, in particular when used in the Wittig conditions.

When step (i) is performed according to the Wittig-Horner reaction, i.e. implementing compounds of formula (b') and (b") as defined above, the obtained compounds having nitrile or ester groups can be hydro lyzed for forming the corresponding carboxylic acid derivative of formula (c).

Compound of formula (b') and of formula (b") may in particular be chosen from (3-cyano)triphenylphosphonium bromide of formula (b'1) 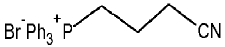 or (b"1) 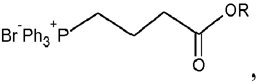 wherein R is a (C₁-C₆)alkyl group and more particularly a methyl or ethyl group in particular when used in the Wittig conditions.

The previous reaction conditions in connection to the Wittig reaction as described above regarding time of reaction, temperature, molar ratio and further purification steps are equally applicable to the Wittig-Horner reaction.

### Step (ii)

According to a particular embodiment, the hydrogenation step (ii) may be performed by using a reducing agent selected from hydrogen, formic acid and its salts (such as sodium formate, ammonium formate, triethylammonium formate, hydrazinium monoformate), alcohols (such as ethanol), amines, hydrocarbons, cyclic olefins (such as cyclohexene, cyclohexadiene), hydrazine, phosphinic acid and phosphinates, phosphorus acid and phosphites, hydrazine, hydrides of boron, aluminum, silicon and tin and diimide and the like, and in particular by using hydrogen.

According to a particular embodiment, step (ii) may be carried out by using a catalyst, namely a metallic catalyst. Said catalyst may be selected from palladium on carbon (Pd/C), platinum on carbon (Pt/C), Raney nickel (Ni), Rhodium on carbon (Rh/C), platinum dioxide and more particularly platinum dioxide anhydrous (PtO₂), palladium hydroxide (Pd(OH)₂), calcium palladium carbonate (Pd/CaCO₃), Rhodium bis(triphenylphosphine) chloride ((PPh₃)₂RhCl), palladium on activated alumina (Pd/Al₂O₃) and rhodium on activated alumina (Rh/Al₂O₃) and the like, and is in particular platinum on carbon.

Still according to said particular embodiment, said step (ii) may be carried out in a solvent, and more particularly an organic solvent which may be selected from toluene, xylene, ethyl acetate, alcohols, tetrahydrofuran, 2-methyltetrahydrofuran, methyl t-butyl ether, dichloromethane, and acetic acid and the like, and is in particular toluene.

Step (ii) may be carried out under inert atmosphere, and in particular under nitrogen atmosphere. The hydrogenation may be performed under pressure, and in particular between 0.5 and 7.0 kg/cm², more particularly between 2.0 and 5.0 kg/cm² and even more particularly between 3.0 and 3.5 kg/cm² (2.94x10⁵ Pa and 3.44x10⁵ Pa).

Step (ii) may be carried out over a wide temperature range which does not cause side reactions but may be carried out at a reaction temperature ranging from 0 °C to reflux temperature, in particular from 10°C to 50°C, and most particularly at ambient temperature.

Step (ii) may be carried out, in particular under hydrogen pressure, for 1 to 48 hours, in particular for 5 to 24 hours, and even more particularly for 11 to 13 hours.

Said step (ii) may be followed by any purification step.

The catalyst may for example be removed, in particular by filtration on Celite, filtration on silica, filtration on cellulose pads or asbestos pad and the like.

During said step (ii), solvents may be removed by concentration under reduced pressure.

### Step (iii)

According to a particular embodiment, cyclization step (iii) may be carried out under intra-molecular cyclization *via* a first stage also named as activation stage with an activating agent, for example with the corresponding acid chloride and the like, and in particular thionyl chloride (SOCl₂) and *via* a second stage, also named Friedel-Craft's acylation.

Under such intra-molecular cyclization conditions, according to a particular embodiment, the activating agent used during the first stage of step (iii) may be selected from thionyl chloride, oxalyl chloride, phosphorus pentachloride (PCl₅) and trifluoroacetic anhydride (TFAA) and the like, and may in particular be thionyl chloride.

Said first stage of step (iii) may be carried out over a wide temperature range which does not cause side reactions, but may be carried out at a reaction temperature ranging from 0°C to reflux temperature, in particular from 15°C to 50°C, and most particularly at ambient temperature. Said first stage of (ii) may further be carried out for 15 min. to 10 hours, in particular for 30 min. to 5 hours, and even more particularly for 1 to 3 hours.

Still according to said particular embodiment, said first stage of step (iii) may be carried out with or without a solvent, and more particularly an organic solvent which may be selected from dichloromethane, chlorobenzene, toluene, dichloroethane, and the like, and is in particular dichloromethane.

Under such intra-molecular cyclization conditions, the second stage of step (iii) may be carried out in the presence of a catalyst. Said catalyst may be selected from aluminum chloride, FeCl₃, InCl₃, GaCl₃, ZnCl₂, CuCl₂, NiCl₂ gallium triflate, tin tetrachloride (SnCl₄), boron trifluoride (BF₃), boron tribromide (BBr₃), boron trichloride (BCl₃) and Tris(pentafluorophenyl)borane and the like and is in particular aluminum chloride.

Still according to said particular embodiment, said second stage of step (iii) may be carried out in a solvent, and more particularly an organic solvent which may be selected from dichloromethane, chlorobenzene, toluene, dichloroethane, and the like, and is in particular dichloromethane.

Still under such intra-molecular cyclization conditions, according to a particular embodiment, said second stage of step (iii) may be carried out over a wide temperature range which does not cause side reactions, but may be carried out at a reaction temperature ranging from 0°C to reflux temperature, in particular from 5°C to 30°C, and most particularly from 10 to 15°C. Said second stage of (ii) may further be carried out for 15 minutes to 10 hours, in particular for 30 minutes to 5 hours, and even more particularly for 1 to 3 hours.

Cyclization step (iii) may alternatively be performed according to any known conditions for achieving such a cyclization.

Thus, according to another particular embodiment, cyclization of compound of formula (d) can also be performed in one stage by direct cyclization of the carboxylic acid. Said direct cyclization step may be performed by using either polyphosphoric acid or TFAA by using additives such as phosphoric acid, trifluoroacetic acid, acetic acid and the like.Under such condition, the following reagents may then more particularly be used: polyphosphoric acid (PPA)/chlorobenzene, P₂O₅/H₃PO₄, methanesulfonic acid/P₂O₅ (Eaton's reagent), trifluoromethanesulfonic acid or trifluoroacetic acid (TFA)/trifluoroacetic anhydride (TFAA) and the like.

Said step (iii) may be followed by any purification step. The pH of the aqueous layer obtained after the second stage as described above may be adjusted, for example by adding hydrochloric acid and said aqueous layer may then be extracted, for example with dichloromethane. The obtained organic phase may then be washed, for example with a saturated bicarbonate solution and/or with saturated sodium chloride solution and the like. During said step (iii), solvents may be removed by additional distillation steps or concentration under reduced pressure.

### Step (iv)

According to a particular embodiment, step (iv) may be carried out by using a catalyst. Said catalyst may be selected from a palladium catalyst, and namely Pd(OAc)₂, 1,1'-bis(diphenylphosphino)ferrocene]dichloropalladium(II) (PdCl₂dppf), tetrakis(triphenylphosphine)palladium(0) (PdP(Ph₃)₄), PdCl₂/binaphtyl ligand and Pd(OAc)₂/bis-diphenylphosphinopropane and the like. The catalyst is in particular Pd(OAc)₂.

According to a particular embodiment, step (iv) may be carried out by using a ligand such as tertiary phosphines. Said ligand may be selected from Xantphos, P(t-Bu)₃.HBF₄, 1,2-bis(diphenylphosphino)-ethane (dppe), 1,3-bis(diphenylphosphino)propane (dppp), bis(diphenylphosphino)xylene (dppx), *(S)*-2,2'-bis(diphenylphosphino)-1,1'-binaphtyle ((S)-BINAP), 2,2'-bis[di-phenylphosphino]methyl-1,1'-biphenyl (BISBI), bis[(2-diphenylphosphino)phenyl] ether (DPEPhos), 1,1'-ferrocenediyl-bis(diphenylphosphine) (dppf), tri(*o*-tolyl)phosphine and the like.

According to another embodiment, said second stage of step (iv) may be carried out in a solvent, and more particularly an organic solvent which may be selected from methanol, N,N-dimethylformamide (DMF), ethyl acetate (AcOEt); acetonitrile, dimethyl sulfoxide (DMSO), acetonitrile, tetrahydrofuran and dichloromethane and the like, and is in particular methanol.

A base may be used for caring out step (iv), which may be selected from triethylamine (Et₃N), *N*,*N-*dicyclohexylmethylamine, carbonates, alkoxides, acetates and a Hünig base and the like. Said base may in particular be triethylamine.

Step (iv) may be performed under carbon monoxide or in presence of any alternative liquid or solid carbon monoxide (CO) sources. As liquid or solid carbon monoxide (CO) source, oxalate derivatives such as dialkyl oxalates, oxalate monoester salts, formate derivatives may be cited such as methyl formate, N-formyl-saccharine, trichlorophenyl formate or hydroxysuccinimide formate. Step (iv) may be performed under pressure, and in particular between 1.0 and 7.0 kg/cm², more particularly between 2.0 and 5.0 kg/cm² and even more particularly between 3.0 and 3.5 kg/cm² (2.94x10⁵ Pa and 3.44x10⁵ Pa).

According to a particular embodiment, said step (iv) may be carried out over a wide temperature range which does not cause side reactions, but may be carried out at a reaction temperature ranging from 20°C to 100°C, in particular from 40°C to 85°C, and most particularly from 60 to 70°C. Said step (iv) may further be carried out for 1 to 48 hours, in particular for 3 to 24 hours, and even more particularly for 5 to 7 hours.

Hereinafter, the present invention will be described in more details, with reference to the following examples. These examples are provided to illustrate the present invention and should not be construed as limiting the scope and spirit of the present invention.

### EXAMPLES

The ¹H NMR spectra are recorded on a Bruker Avance 400 spectrometer, with the chemical shifts (δ in ppm) using the solvent either CDCl₃ referenced at 7.28 ppm or DMSO-d₆ referenced at 2.54 ppm at a temperature of 295 K.

The liquid chromatography data are obtained on an Agilent UPLC instrument equipped with a Photo Diode Array (PDA) detector. The analysis is carried out on Waters X-select C18, 150 x 4.6 mm, 3.5 µm column. Mobile phase A consists of 0.1% trifluoroacetic acid in water and Mobile phase B consists of 0.1% trifluoroacetic acid in acetonitrile : Methanol (90 : 10). Program gradient elution (T/% B =0.01/5, 6/30, 12/60, 16/90, 34/90, 35/5, 40/5) is used with UV detection at 210 nm at a flow rate of 1.0 mL per min. The column temperature is maintained at ambient conditions. The data is recorded using Waters Empower software [Run time - 40 min].

Purity by HPLC as reported herein after is expressed as percentage area (a/a).

### Example 1: Compound of formula (c) 5-(3-bromophenyl)pent-4-enoic acid

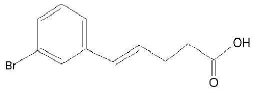

To a solution of (3-carboxypropyl)triphenylphosphonium bromide (b) (139.1 g, 0.324 mol.) in THF (300 mL), was added sodium methoxide (62.5 g, 1.157 mol.) followed by a solution of 3-bromobenzaldehyde (a) (50.0 g, 0.270 mol.) in THF (100 mL) at 40 to 45 °C. The reaction mixture turned pink and was stirred at 40 to 45 °C for 10 hours. After completion of the reaction, the mixture was allowed to room temperature, quenched with water (600 mL), solvent (THF) was removed by distillation and the resulted aqueous layer was washed with dichloromethane (CH₂Cl₂) (3 x 250 mL) to remove triphenylphosphine oxide (Ph₃P=O). The pH of the aqueous layer is adjusted to ∼1 using 6.0 N hydrochloric acid solution and extracted with toluene (3 x 250 mL). Gathered organic phases were washed with saturated sodium chloride solution (250 mL) and solvent was concentrated under reduced pressure. The obtained material was proceeded to next stage without any further purification (62.5 g, Yield: 90.0%, Purity by HPLC: 99.0% a/a including both the isomers). ¹H NMR (400 MHz, CDCl₃): δ 11.15 (br s,1H), 7.48 (s, 1H), 7.32-7.34 (d, J= 6.15 Hz, 1H), 7.23-7.25 (d, J= 6.67 Hz, 1H), 7.14-7.17 (t, J= 7.12 Hz, 1H), 6.35-6.39 (m, 1H), 6.19-6.23 (m, 1H), 2.47-2.64 (m, 4H) ppm.

### Example 2: Compound of formula (d) 5-(3-Bromophenyl)pentaenoic acid.

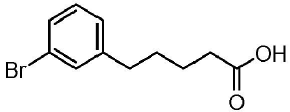

The reaction was carried out in a 2.0 L stainless steel autoclave equipped with a mechanical stirrer. The reagents were charged under nitrogen atmosphere in the following order: Toluene (500 mL), 5-(3-bromophenyl)pent-4-enoic acid (c) (50 g, 0.196 mol.) and 10% Pd/C (50% wet, 5.0 g, 10% w/w). The air in the autoclave was replaced with nitrogen followed by hydrogen and the hydrogen pressure adjusted to 3.0 to 3.5 kg/cm². The mixture was stirred for 12 hours at room temperature by maintaining the hydrogen pressure at 3.0 to 3.5 kg/cm². The catalyst was removed by filtration over Celite and the solvent was concentrated under reduced pressure to give 5-(3-bromophenyl)pentanoic acid (48.5 g, Yield: 96%, Purity by HPLC: 96% a/a). ¹H NMR (400 MHz, DMSO-d₆): δ 12.0 (br s, 1H), 7.36-7.41 (m, 2H), 7.17- 7.26 (m, 2H), 2.56-2.60 (t, 2H), 2.21-2.25 (t, 2H), 1.46-1.6 (m, 4H) ppm.

### Example 3: Compound of formula (e) 7-Bromo-1-benzosuberone

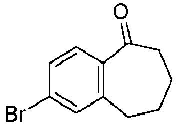

To a solution of 5-(3-bromophenyl)pentanoic acid (d) (100 g, 0.388 mol.) in dichloromethane (CH₂Cl₂) (800 mL), was added thionyl chloride (SOCl₂) (92.54 g, 0.778 mol.) and stirred for 2 hours at room temperature. After completion of reaction, the mixture was concentrated under reduced pressure at below 45 °C. The resulted residue was dissolved in dichloromethane (CH₂Cl₂) (300 mL) and was slowly added to a precooled mixture of aluminum chloride (77.8 g, 0.583 mol.) in dichloromethane (CH₂Cl₂) (250 mL) at below 15 °C and stirred for 2 hours at 10 to 15 °C. The mixture was quenched into 1.0 N hydrochloric acid solution (1000 mL), organic layer was separated and resulted aqueous layer was extracted with dichloromethane (CH₂Cl₂) (500 mL). The combined organic layer was washed sequentially with water (500 mL), saturated bicarbonate solution (500 mL) and finally with saturated sodium chloride solution (500 mL). The solvent was concentrated under reduced pressure to obtain 7-bromo-1-benzosuberone as a brown solid. (91.5 g, Yield: 98%, Purity by HPLC: 90% a/a).¹H NMR (400 MHz, CDCl₃): 7.58-7.61 (d, J = 8.3 Hz, 1H), 7.43-7.45 (d, J = 8.2 Hz, 1H), 7.38-7.39 (d, J=1.8 Hz, 1H), 2.88-2.91 (t, J = 6.0 Hz, 2H), 2.71-2.74 (t, J = 6.2 Hz, 2H), 1.77-1.92 (m, 4H) ppm.

### Example 4: Compound of formula (f) Methyl 1-benzosuberone-7-carboxylate

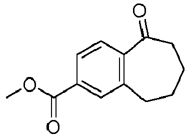

The reaction was carried out in a 2.0 L stainless steel autoclave equipped with a mechanical stirrer. The reagents were charged in the following order: methanol (500 mL), 7-bromo-1-benzosuberone (e) (100 g, 0.418 mol.), Xantphos (4.8 g, 8.3 mmol.), Pd(OAc)₂ (1.3 g, 5.8 mmol.) and Et₃N (63.47 g, 0.627 mol.). The air in the autoclave was replaced with carbon monoxide and the pressure adjusted to 3.0 to 3.5 kg/cm². The mixture is heated to 65 to 70 °C by maintaining the carbon monoxide pressure at 3.0 to 3.5 kg/cm² for 6 hours. After completion of the reaction, the mixture was allowed to room temperature, filtered over Celite and the solvent was concentrated under reduced pressure. The obtained residue was triturated with water (500 mL) followed by heptane (400 mL) at room temperature to afford methyl 1-benzosuberone-7-carboxylate as a yellow colored solid (59.6 g, Yield: 65%, Purity by HPLC: 99.5% a/a). ¹H NMR (400 MHz, CDCl₃): δ 7.93-7.96 (m, 1H), 7.89 (s, 1H), 7.73-7.75 (d, J=7.9, 1H), 3.94 (s, 3H), 2.96-3.0 (m, 2H), 2.74-2.77 (m, 2H), 1.87-1.94 (m, 2H),1.79-1.85 (m,2H) ppm.

## Claims

1. A process for preparing compound (A5) 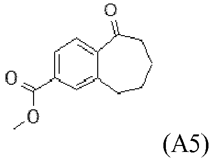 **characterized in that** a compound of formula (e) 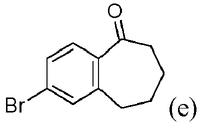 is alkoxycarbonylated to prepare compound (A5).

2. The process according to claim 1, **characterized in that** compound of formula (e) is prepared by cyclizing of compound of formula (d)

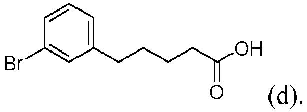

3. The process according to claim 2, **characterized in that** compound of formula (d) is prepared by hydrogenating compound of formula (c)

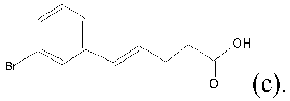

4. The process according to claim 3, **characterized in that** compound of formula (c) is prepared by reacting a compound of formula (a) 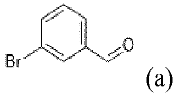 with a compound of formula 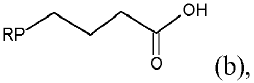 of formula 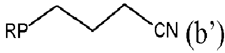 or of formula 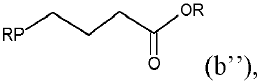 wherein RP means a X⁻P⁺R'₃ group or a P(=O)(OR")₂, R means a (C₁-C₆)alkyl group, respectively under Wittig or Wittig-Horner conditions, R' means a (C₁-C₃)alkyl group or a phenyl group, R" means a (C₁-C₃)alkyl group and X is an halogen atom, and in particular a bromine atom, the carbon in alpha of the phosphorus atom being a carbanion.

5. The process for preparing compound (A5) according to claim 1, **characterized in that** it comprises:
- a step **(i)**: reacting a compound of formula (a) 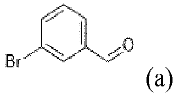 with a compound of formula 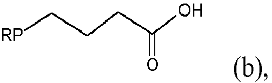 of formula 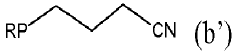 or of formula 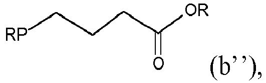 wherein RP means a X⁻P⁺R'₃ group or a P(=O)(OR")₂, respectively under Wittig or Wittig-Horner conditions, R means a (C₁-C₆)alkyl group, R' means a (C₁-C₃)alkyl group or a phenyl group, R" means a (C₁-C₃)alkyl group and X is an halogen atom, and in particular a bromine atom, the carbon in alpha of the phosphorus atom being a carbanion,
to prepare compound of formula (c) 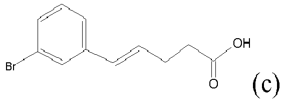
- a step **(ii)**: hydrogenating compound of formula (c) to prepare compound of formula (d) 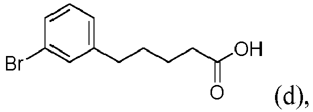
- a step **(iii)**: cyclizing of compound of formula (d) to prepare compound of formula (e) 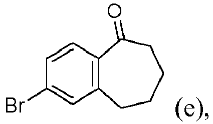 and
- a step **(iv)**: alkoxycarbonylating of compound of formula (e) to prepare compound (A5).

6. The process according to anyone of the preceding claims, **characterized in that** the alkoxycarbonylation or step **(iv)** as defined in claim 5 is carried out by using a catalyst, which catalyst may be selected from a palladium catalyst, in particular selected from Pd(OAc)₂, 1,1'-bis(diphenylphosphino)ferrocene]dichloropalladium(II), tetrakis(triphenylphosphine)palladium(0), PdCl₂/binaphtyl ligand and Pd(OAc)₂/bis-diphenylphosphinopropane, and more particularly is Pd(OAc)₂.

7. The process according to anyone of the preceding claims, **characterized in that** the alkoxycarbonylation or step **(iv)** as defined in claim 5 is carried by using a ligand, which ligand may be Xantphos, P(t-Bu)₃.HBF₄, 1,2-bis(diphenylphosphino)-ethane, 1,3-bis(diphenylphosphino)propane, bis(diphenylphosphino)xylene, *(S)*-2,2'-bis(diphenylphosphino)-1,1'-binaphtyle, 2,2'-bis[di-phenylphosphino]methyl-1,1'-biphenyl, bis[(2-diphenylphosphino)phenyl] ether, tri(*o*-tolyl)phosphine or 1,1'-ferrocenediyl-bis(diphenylphosphine).

8. The process according to anyone of the preceding claims, **characterized in that** the alkoxycarbonylation or step (iv) as defined in claim 5 is carried out by using a base, which base may be selected from triethylamine, *N*,*N*-dicyclohexylmethylamine, carbonates, alkoxides, acetates and a Hünig base, and is in particular triethylamine.

9. The process according to anyone of claims 2 to 8, **characterized in that** the cyclization or step **(iii)** as defined in claim 5 is carried out in a first activation stage with an activating agent, which may be selected from thionyl chloride, oxalyl chloride; phosphorus pentachloride and trifluoroacetic anhydride, and may in particular be thionyl chloride and in a second stage in the presence of a catalyst which catalyst may be selected from aluminum chloride, FeCl₃, InCl₃, GaCl₃, ZnCl₂, CuCl₂, NiCl₂, gallium triflate, tin tetrachloride, boron trifluoride, boron tribromide, boron trichloride and Tris(pentafluorophenyl)borane, and is in particular aluminum chloride.

10. The process according to anyone of claims 3 to 9, **characterized in that** the hydrogenation or step **(ii)** as defined in claim 5 is performed by using a reducing agent selected from hydrogen; formic acid and its salts, such as sodium formate, ammonium formate, triethylammonium formate, hydrazinium mono formate; alcohols, such as ethanol; amines; hydrocarbons; cyclic olefins, such as cyclohexene, cyclohexadiene; hydrazine, phosphinic acid and phosphinates; phosphorus acid and phosphites; hydrazine; hydrides of boron; aluminum; silicon and tin and diimide and in particular by using hydrogen.

11. The process according to anyone of claims 3 to 10, **characterized in that** the hydrogenation or step **(ii)** as defined in claim 5 is carried out by using a catalyst which may be selected from palladium on carbon, platinum on carbon, Raney nickel, Rhodium on carbon, platinum dioxide and more particularly platinum dioxide anhydrous, palladium hydroxide, calcium palladium carbonate, Rhodium bis(triphenylphosphine) chloride, palladium on activated alumina and rhodium on activated alumina, and is in particular platinum on carbon.

12. The process according to claim 4 or 5, **characterized in that** the reaction of compound of formula (a) with compound of formula (b), (b') or (b") as defined in claim 4 or step **(i)** as defined in claim 5 is carried out in the presence of a base which may be selected from Li/Na/K alkoxides, and more particularly sodium methoxide, Li/Na/K bis(trimethylsilyl)amide, Li/Na/K diisopropylamide, NaH, tBuOK and n-BuLi and is in particular sodium methoxide.

13. The process according to anyone of claim 4, 5 or 12, **characterized in that** the reaction of compound of formula (a) with compound of formula (b), (b') or (b") as defined in claim 4 or step **(i)** as defined in claim 5 is carried out in a solvent, and more particularly an organic solvent which may be selected from tetrahydrofuran, ethers such as dimethylether, methylated tetrahydrofuran, toluene, dimethyl sulfoxide and dichloromethane as well as their mixture, each of said solvent being optionally mixed with methanol, and may in particular be tetrahydrofuran optionally mixed with methanol.

14. The process according to anyone of claim 4, 5, 12 or 13, **characterized in that** compound of formula (b) as defined in claim 4 is selected from (3-carboxypropyl)triphenylphosphonium bromide, 4-(diethoxyphosphoryl)butanoic acid, 4-(dimethoxythiophosphoryl)butanoic acid, compound of formula (b') as defined in claim 4 is (3-cyano)triphenylphosphonium bromide and compound of formula (b") as defined in claim 4 is 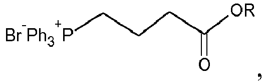 wherein R is a (C₁-C₆)alkyl group and more particularly a methyl or ethyl group.

15. A process for the preparation of compound (1) or one of its pharmaceutically acceptable salts 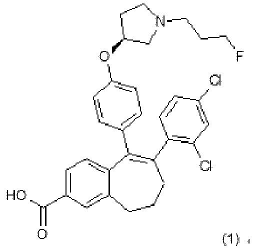 **characterized in that** it comprises the step of using a compound (A5) as prepared by the process according to anyone of the preceding claims.

16. The process for preparing a compound (1) according to the preceding claims, further comprising the step of preparing a pharmaceutical composition containing such compound (1), with pharmaceutically acceptable excipients.
